# EUROPÄISCHE PATENTSCHRIFT

(11) **EP 0 914 602 B1**
(45) Veröffentlichungstag und Bekanntmachung des Hinweises auf die Patenterteilung: **14.11.2001**
(21) Anmeldenummer: 97936558.2
(22) Anmeldetag: 25.07.1997
(51) Int. Cl.: G01N 21/82, G01N 21/51

(54) **VERFAHREN UND EINRICHTUNG ZUR BESTIMMUNG DER EXTINKTION EINER LICHTSTRAHLUNG BEIM DURCHDRINGEN EINER PROBE**
PROCESS AND DEVICE FOR DETECTING THE EXTINCTION OF A LIGHT BEAM WHILE CROSSING A SAMPLE
PROCEDE ET DISPOSITIF POUR DETERMINER L'EXTINCTION D'UN RAYON LUMINEUX LORSQUE CELUI-CI EST TRAVERSE PAR UN ECHANTILLON

(30) Priorität: 25.07.1996 DE 19629992
(43) Veröffentlichungstag der Anmeldung: 12.05.1999
(73) Patentinhaber: Tarotest GmbH, 01855 Sebnitz (DE)
(72) Erfinder: Winkler, Manfred, 01855 Sebnitz (DE)
(74) Vertreter: Pätzelt, Peter, Dipl.-Ing.
(86) Internationale Anmeldenummer: DE9701574
(87) Internationale Veröffentlichungsnummer: WO9804905

(56) Entgegenhaltungen:
- EP-A- 0 178 910
- EP-A- 0 364 583
- EP-A- 0 414 224
- WO-A-90/08961
- GB-A- 2 069 692
- US-A- 3 847 482
- US-A- 4 118 625
- US-A- 5 502 651

## Beschreibung

Die Erfindung betrifft ein Verfahren und zwei Einrichtungen zur Bestimmung der Extinktion oder Transmission einer Lichtstrahlung beim Durchdringen einer Probe nach den Oberbegriffen der Ansprüche 1 bzw. 5 oder 6.

Nach dem Stand der Technik ist die Bestimmung der Extinktion bzw. Transmission einer Lichtstrahlung durch eine Probe in vielfältiger Form bekannt. Dabei ist es grundsätzlich gleichgültig, ob letztendlich die Extinktion oder die Transmission als solche zur Auswertung gelangen, da beide alternative Werte sind. In der Folge wird somit vereinfachend überwiegend von der Extinktion gesprochen.

Die Bestimmung der Extinktion betrifft regelmäßig eine Probe, die aus einem transparenten Probenbehälter mit einer darin befindlichen und zu untersuchenden Flüssigkeit besteht. Der Probenbehälter kann auch ein in einer Einrichtung integriertes Konstruktionsteil sein. Die Probe selbst ist meist eine Flüssigkeit, kann aber auch ein Flüssigkeits- Gasgemisch sein. Meist ist die Probe eine zu untersuchende Flüssigkeit als erstes Reagenz, die mit einem zweiten Reagenz reagiert, wobei das Resultat der Reaktion zur Auswertung gelangt. Häufig ist das erste Reagenz mit einer neutralen Flüssigkeit verdünnt, damit eine höhere Basis-Transmission die Bestimmung der Extinktion erleichtert.

Die praktische Anwendung derartiger Verfahren und Einrichtungen erfolgt in großer Breite im medizinischen und veterinärmedizinischen Bereich, bei der Lebensmittelüberwachung, der Überwachung im Umweltbereich (im besonderen Maße für Wasser), bei der Systemüberwachung in chemisch und/oder physikalisch reagierenden Anwendungen für die Industrie und den Haushalt, z. B. bei Waschmaschinen und Spülmaschinen.

Die DE 44 07 332 A1 beschreibt z. B. ein Verfahren zur Bestimmung der Extinktion oder Transmission, bei dem die von einer breitbandigen Lichtquelle ausgesandte Strahlung geteilt wird. Ein Teil der Strahlung wird über einen ersten, einen Meßkanal bildenden Lichtweg durch die Probe und ein die Meßwellenlänge oder Polarisationsrichtung ausfilterndes, auswechselbares erstes optisches Filter zu einem ersten Photoempfänger geleitet. Ein weiterer Teil der Strahlung der Lichtquelle wird über einen zweiten, einen Referenzkanal bildenden Lichtweg, der insbesondere ein zweites optisches Filter aufweist zu einem zweiten Photoempfänger geleitet. Der Strahlungsintensitätswert im Referenzkanal wird unter konstanten optischen Bedingungen, insbesondere mittels eines festen optischen Filters, für mindestens eine andere Wellenlänge bzw. einen anderen Wellenlängenbereich oder einen anderen Polarisationszustand innerhalb des Strahlungsspektrums der breitbandigen Lichtquelle als der mittels des auswechselbaren Filters im Meßkanal ermittelte Strahlungsintensitätswert ermittelt. Der im Referenzkanal ermittelte Strahlungsintensitätswert wird dann noch zusätzlich um einen Wert korrigiert, der für die durch das auswechselbare Filter im Meßkanal ausgefilterte Wellenlänge oder Polarisationsrichtung in Abhängigkeit von der Lichtquelle und den konstanten optischen Bedingungen im Referenzkanal vorbestimmt ist. Mit dieser Lösung soll der zuvor bekannte Stand der Technik vereinfacht werden. Insbesondere soll der Filterwechsel im Referenzkanal bei abweichenden Wellenlängen vermieden und die Kosten für die Photometerausrüstung erheblich verringert werden.
Diese beschriebene Veröffentlichung verkörpert im wesentlichen den relevanten Stand der Technik für die vorliegende Erfindung.

Der Stand der Technik geht dabei von der grundsätzlichen Erkenntnis aus, daß zur Gestaltung optischer Geräte bzw. für eine nutzbare optische Messung immer ein gefiltertes Licht eines bestimmten eingegrenzten Wellenlängenbereiches erforderlich ist. Bei der Bestimmung der Extinktion geht der Fachmann des weiteren davon aus, daß es grundsätzlich erforderlich ist, den Strahlungswert einer Referenzstrahlung zu bestimmen, der ohne den Durchgang durch die Probe einen Photoempfänger erreicht. Diese Referenzstrahlung wird als Grundlage für die Bestimmung der Extinktion der Lichtstrahlung durch die Probe genutzt. Die Folge ist immer ein relativ aufwendiges optisches Meßsystem.

Ein andersartiger optischer Sensor wird in der DE 4336520 A1 für die Analyse von Waschlauge angegeben. Der Sensor weist ein Meßrohr auf, das im Bereich des ein- und austretenden Strahlenbündels einer Licht emittierenden Strahlungsquelle jeweils leicht kugelförmig nach innen gewölbt ist. Wassergefüllt wirkt das Meßrohr als schwache Zersträuungslinse, die das Strahlenbündel im Bereich des Fototransistors aufweitet. Das mit Luft gefüllte Meßrohr läßt die Strahlung ungebeugt zum Fototransistor gelangen. Die abweichenden Strahlungswerte werden zur Auswertung genutzt.

Der Erfindung liegt damit als Aufgabe zugrunde, ein Verfahren und eine Einrichtung zur Bestimmung der Extinktion einer Lichtstrahlung durch eine Probe mit einer zu untersuchenden Flüssigkeit der eingangs genannten Art anzugeben, die den technischen Aufwand für die optische und meßtechnische Ausrüstung wesentlich verringert, ohne daß sich die Genauigkeit der Messung verschlechtert.

Des weiteren besteht die Aufgabe darin, eine vorteilhafte Anwendung anzugeben.

Die Aufgabe wird erfindungsgemäß durch ein Verfahren gelöst, das die Merkmale des Anspruchs 1 aufweist bzw. zwei Einrichtungen mit den Merkmalen der Ansprüche 5 und 6. Weiterbildungen der Erfindung sind in den Unteransprüchen gekennzeichnet.

Der Lösung für das erfindungsgemäße Verfahren sowie die Einrichtung zur Bestimmung der Extinktion liegt dabei der Gedanke zugrunde, daß für die qualitativen und/oder quantitativen Aussagen über die Eigenschaften einer zu untersuchenden Probe, die als erstes Reagenz mit einem zweiten Reagenz reagiert, die Veränderung der Extinktion verwendet wird. Nach dem Stand der Technik werden demgegenüber grundsätzlich die absoluten Extinktionswerte einer Auswertung zugrunde gelegt.

Zur erfindungsgemäßen Bestimmung der Veränderung der Extinktion werden vor dem Beginn der Reaktion der Reagenzien oder unmittelbar nach dem Beginn, d. h. nach dem Zusetzen des zweiten Reagenz zur Probe als erstes Reagenz, ein erster Extinktionswert bestimmt und danach wird der Fortschritt der Extinktion bzw. das Ende der Extinktion ermittelt. Die Differenzen der jeweils ermittelten Extinktionswerte werden dann elektronisch mit vorbestimmten Referenzwerten verglichen und die ermittelten Abweichungen für die qualitativen und/oder quantitativen Aussagen über die Eigenschaften der untersuchten Probe genutzt. Die Erfindung weist keine gesonderten Filter auf. Die Probe selbst wirkt wie jeder lichtdurchlässige Körper als Filter. Es werden immer nur die Lichtwerte gemessen, die die Probe durchdrungen haben und den Lichtsensor erreichen.

Dabei kann die Zusammenführung der Reagenzien variieren, indem sich das erste Reagenz in dem Probenbehälter befindet und das zweite Reagenz hinzugefügt wird oder umgekehrt.

Vielfach wird das erste Reagenz in einer neutralen Flüssigkeit verdünnt.

Die neutrale Flüssigkeit kann aber auch ein Basisreagenz sein, welches mit einem Startreagenz aktiviert wird und danach erst die Reaktion mit dem ersten Reagenz beginnt.

Basisreagenz oder Startreagenz können in ihrer Wirkung auch auf die Katalyse beschränkt sein.

Alle chemischen und biochemischen Reaktionen, die zu einer Farbänderung führen, absorbieren einen Teil des auftreffenden Lichtes und zwar regelmäßig proportional zur Konzentration eines der Stoffe, die die Reaktion zur Farbbildung hervorrufen. In wenigen Fällen wird das Licht exponentiell absorbiert.

Von besonderem Vorteil ist auch eine gezielte Auswahl der Leuchtdiode nach Anspruch 8 und des Lichtsensors nach Anspruch 9. Damit kann in sehr vorteilhafter Weise das abgestrahlte Licht bzw. das erfaßte Licht auf die Wellenlängen eingeschränkt werden, bei welchen die Extinktion einer spezifischen Probe am stärksten differenziert erfaßt werden kann. Es gibt eine Vielzahl von Wellenlängen, die von einer spezifischen Extinktion weniger beeinflußt werden und trotz einer realen Extinktion die Probe weitgehend unbeeinflußt durchdringen. Derartige Wellenlängenbereiche sind bei der Messung uninteressant und besser auszuschließen.

Die praktisch verfügbaren Leuchtdioden weisen meist Bandbreiten der spezifischen Wellenlängen von 50 bis 150 nm auf. Lichtsensoren können auf spezifische Bandbreiten von Wellenlängen zwischen 50 bis 100 nm eingegrenzt werden.

Das Ermitteln der vorbestimmten Referenzwerte erfolgt in der Regel durch Messung der Veränderung der Extinktionswerte bei "normalen" Proben, der ersten Reagenzien mit den spezifischen zweiten Reagenzien. "Normal" ist das, was zulässig ist, im medizinischen Bereich ist beispielsweise ein "gesundes" Reagenz "normal". D.h. alle Abweichungen gegenüber diesen Referenzwerten, die aus einer bestimmten Toleranzbreite herausfallen, sind einer Beurteilung des Sachverhaltes, je nachdem um was für eine Probe es sich handelt, zu unterziehen.

Vorteilhafte Weiterbildungen der Erfindung sind in den Unteransprüchen gekennzeichnet bzw. werden nachstehend zusammen mit der Beschreibung der bevorzugten Ausführung der Erfindung näher dargestellt.

Die Erfindung wird nachstehend zuerst an einer erfindungsgemäßen Einrichtung und danach deren Einsatz an zwei Ausführungsbeispielen näher erläutert.

Die zugehörige Zeichnung zeigt eine schematische Darstellung der erfindungsgemäßen Einrichtung.
Die Einrichtung besteht im zentralen Bereich aus einem Lichtschacht 1, in dem sich quer zur Längsachse, hier vertikal, ein Meßschacht 2 befindet. Der Meßschacht 2 ist paßgenau mit einem Probenbehälter 3 abgestimmt, derart daß der Probenbehälter 3 in Form einer Küvette leicht und unter Vermeidung der Möglichkeit, daß Licht unkontrolliert an der Küvette vorbeidiffundieren kann, in den Meßschacht 2 eingesetzt werden kann. An einem Ende des Meßschachtes 2 befindet sich als Lichtquelle 4 eine Leuchtdiode, die beispielsweise eine Bandbreite der Wellenlänge des emittierten Lichtes zwischen 50 und 150 nm aufweist. Der verwendete tatsächliche Spektralbereich einer konkreten Lichtquelle 4 wird vorteilhaft an die konkret zu untersuchende Probe angepaßt.

Am gegenüberliegenden Ende des Lichtschachtes 1 befindet sich ein Lichtsensor 5. Das Licht, welches von der Lichtquelle 4 abgestrahlt wird und in den Lichtschacht 1 gelangt, durchdringt im Bereich des Meßschachtes 2 den Probenbehälter 3 mit der darin befindlichen Probe und wird dabei zwangsläufig mit einem konstanten Faktor gefiltert und mit einem veränderlichen Faktor absorbiert. Selbstverständlich ist darauf zu achten, daß der Probenbehälter 3 so gefüllt ist, daß die Transmission des Lichtes nicht durch Lufteinschlüsse oder einen zu geringen Flüssigkeitsspiegel beeinflußt wird.

Der konstante Faktor zur Lichtfilterung wird maßgeblich vom Material des Probenbehälters beeinflußt und in einem bestimmten Maße auch von der jeweiligen flüssigen Probe.

Die Lichtmenge, welche ungefiltert den Probenbehälter 3 durchdringt, weist damit die maximale Breite des von der Lichtquelle 4 abgestrahlten Spektrums auf. D. h. mit der erfindungsgemäßen Einrichtung, ohne einen nach dem Stand der Technik vorgeschalteten speziellen Filter, ist es möglich, eine maximale Lichtmenge dem Lichtsensor 5 zuzuführen. Mit dieser wesentlich höheren transmittierten Lichtmenge zu Beginn der Extinktionsbestimmung bzw. Transmissionsmessungen ist es in der Folge auch möglich, einen wesentlich höheren Extinktionswert bis etwa 3,5 zu ermitteln. Wenn zusätzlich die Lichtquelle 4 gepulst wird, kann die Meßrate bis auf einen Extinktionswert von 4,5 gesteigert werden. Die Ursache liegt dabei darin begründet, daß der Lichtsensor eine gepulste Lichtmenge besser und genauer definieren kann als eine analog veränderte Lichtmenge.
Die Meßsignale des Lichtsensors 5 werden einer Vergleichseinheit 6 zugeführt, in der die Meßsignale digitalisiert zumindestens kurzzeitig gespeichert werden. In der Regel wird vorher aus einem Referenzwertspeicher 7 ein spezieller, die aktuelle Probe betreffender Referenzwert aktiviert und in die Vergleichseinheit 6 eingegeben.

Dabei ist der Referenzwert in der Regel ein "Normalwert", d. h. je nach der zu untersuchenden Probe ist der Normalwert der allgemein übliche normale Wert bzw. die Werte, die für eine zulässige oder auch "gesunde" Probe in entsprechenden Versuchen definiert wurden, vorzugsweise einschließlich einer entsprechenden Differenzwerttabelle.

Innerhalb der Vergleichseinheit 6 werden in der Folge die von dem Lichtsensor 5 aktuell gemessenen Extinktionswerte mit den Referenzwerten und der entsprechenden Toleranztabelle verglichen und die Abweichungen werden einer spezifischen Aussage zugeordnet, die von einer Ausgabeeinheit 8 in verschiedener Weise bereitgestellt wird. Die Bereitstellung der Aussage kann über ein Display erfolgen, aber auch über digitale Signalausgaben, die von weiteren Geräten verarbeitet werden oder mittels eines Druckers ausgegeben werden können.

Im Referenzwertspeicher 7 können dabei sehr verschiedene Referenzwerte und zugehörige Differenzwerttabellen gespeichert werden. Das können beispielsweise auf dem Gebiet der Humanmedizin Werte für Bestimmungen des Blutzuckergehaltes oder Enzym- sowie Urinbestimmungen sein. Wesentlich ist lediglich, daß für die jeweiligen zu bestimmenden Proben als erste Reagenzien andere zweite Reagenzien verfügbar sind, die zu einer Extinktion der Probe führen.

Diese erfindungsgemäße Einrichtung kann insbesondere im Auswertebereich auch variiert werden, je nach dem welche spezifische Aufgabe gelöst werden soll.

Nachfolgend werden unter Nutzung der vorbeschriebenen Einrichtung zwei spezifische Ausführungsbeispiele näher beschrieben.

### Ausführungsbeispiel I

Im Ausführungsbeispiel I soll der Blutzuckerwert (Glukose) einer Probe bestimmt werden.

Dazu wird ein Glukose-Bestimmungsreagenz als Basisreagenz, welche später mit einer Startreagenz aktiviert wird und beide in Übereinstimmung mit den Patentansprüchen das zweite Reagenz bilden, in einen Probenbehälter, eine Küvette, gefüllt. Dieses zweite Reagenz ist geeignet, mit der Glukose eines Blutserums, hier die zu untersuchende Flüssigkeit und allgemein als erstes Reagenz bezeichnet, derart zu reagieren, daß die Extinktion der gemischten Flüssigkeit steigt bzw. die Transmission mit zunehmender Zeit abnimmt.

Derartige zweite Reagenzien sind in der Analysentechnik bekannt und verfügbar.

Im Beispiel werden dabei 1200 µl des Glukose-Bestimmungsreagenz als Basisreagenz in den Probenbehälter 3 eingefüllt und diesem 10 µl Blutserum als erstes Reagenz, eine als solche farblose Blutflüssigkeit, zugesetzt und mechanisch leicht geschüttelt und damit vermischt. Diese vorbereitete Probe verhält sich noch neutral bis später das Startreagenz zugesetzt wird. Nach dem Einsetzen dieser vorbereiteten Probe in den Meßschacht 2 wird das Gerät zur Bestimmung der Extinktion in Betrieb gesetzt. Dazu wird mittels einer Eingabetastatur der entsprechende Referenzwert zur Glukose-Bestimmung aus dem Referenzwertspeicher 7 aktiviert und der Vergleichseinheit 6 zugeführt. Die Leuchtdiode wird eingeschaltet und eine erste Transmissionsmessung durchgeführt. Diese Messung stellt gleichzeitig eine Kalibrierung dar, indem eine Ausgangstransmission ermittelt wird.

Zur eigentlichen Bestimmung der Extinktion werden der beschriebenen vorbereiteten Probe 100 µl des Startreagenz zugesetzt und gleichzeitig mit einer periodischen Messung der Transmission begonnen.

Die jeweiligen Werte werden in digitalisierter Form der Vergleichseinheit 6 zugeführt und den bereits aktivierten Referenzwerten gegenübergestellt. Das Ergebnis kann ständig über die Auswerteeinheit auf einem Display angezeigt oder in der Auswerteeinheit gespeichert werden bis die Messung abgeschlossen ist. Das Ende der Messung wird in der Regel dann festgelegt, wenn eine Änderung der Extinktion nicht mehr exakt bestimmt werden kann.

Nach der festgelegten Zeit wird die Messung der Transmissionswerte abgebrochen und aus dem errechneten Wert der Änderung der Extinktion pro Zeiteinheit wird unter Anwendung eines internen Faktors die Aktivität des Enzyms bestimmt.

Die Lichtquelle 4 wird dabei in gepulster Form betrieben, da damit wesentlich genauere und umfassendere Messungen möglich sind. Diese Betriebsart sollte grundsätzlich für alle Messungen gewählt werden. Der elektronische Aufwand zur Realisierung ist dabei sehr gering.

Eine Bestimmung der Extinktion in der beschriebenen Art ist völlig unkompliziert und sehr schnell zu realisieren. Es müssen keine Filter gewechselt werden und das Gerät ist überaus einfach.

### Ausführungsbeispiel II

Im Ausführungsbeispiel II soll die Enzymaktivität von Kreatinin, eine Verbindung, die eine Aussage zur Funktion der Nieren gestattet, im Blutserum bestimmt werden. Dabei wird davon ausgegangen, daß die Referenzwerte noch nicht bekannt sind und diese erst ermittelt werden müssen.

In ähnlicher Weise wie bei dem Ausführungsbeispiel I werden 200 µl Kreatinin-Standard als erstes Reagenz und 1000 µl Reaktionsreagenz als zweites Reagenz vermischt und im Probenbehälter 3 in den Meßschacht 2 eingesetzt.

Vorher wurde über die Eingabetastatur nicht ein vorhandener Referenzwert aktiviert, sondern es wurde über spezifische Befehle die Erstellung eines Referenzwertes aktiviert.

Unmittelbar nach dem Mischen der Probe und Einsetzen des Probenbehälters 3 in den Meßschacht 2 wird die Messung der Transmissionswerte in periodischen Zeiteinheiten eingeleitet. Nach ca. sechs Minuten ist die Bestimmung der Extinktionswerte für die vorliegende Kreatinin-Standard-Probe abgeschlossen und gespeichert. Die Kreatinin-Standard-Pro- be ist dabei eine anerkannte Probe für Kreatinin von einem normalen gesunden Körper. In der Folge werden diese Extinktionswerte als Referenzwerte im Referenzwertspeicher 7 gespeichert.

Nachfolgend können diese so ermittelten Werte zur Bestimmung von Abweichungen von möglichen annormalen Kreatinin-Proben verwendet werden. Im vorliegenden Fall kann natürlich nur die tatsächliche Abweichung ermittelt werden. Wenn die Abweichungen auch in bewerteter Form ausgegeben werden sollen, dann ist eine entsprechende medizinische Transferierung erforderlich.

Bei der Bestimmung der Enzymaktivität einer neuen Kreatinin-Probe wird zuerst der nunmehr im Referenzwertspeicher 7 gespeicherte Referenzwert für Kreatinin aktiviert und es werden in ähnlicher Weise wie vorbeschrieben, 200 µl Kreatinin-Serum als erstes Reagenz in 1000 µl Reaktionsreagenz als zweites Reagenz pipettiert und vermischt.

Die Messung erfolgt in gleicher Weise wie bereits beschrieben und die Abweichungen der aktuellen Werte von den Referenzwerten werden an einem Display angezeigt und an einen angeschlossenen Drucker ausgegeben. Die Bewertung der Messung bleibt, mindestens solange wie keine entsprechenden Kriterien zu den Referenzwerten festgelegt wurden, dem Arzt vorbehalten.

Die Erfindung ist selbstverständlich nicht auf die beschriebene Ausführungsbeispiele beschränkt. So ist es ohne weiteres möglich, zahlreiche weitere Einsatzgebiete zu definieren. Voraussetzung ist lediglich die Möglichkeit, daß der zu untersuchende Stoff (erstes Reagenz) flüssig ist oder in einer Flüssigkeit gelöst werden kann und mit einem anderen (zweiten) Reagenz derart reagiert, daß eine Extinktion bestimmt werden kann. Das zweite Reagenz muß ebenfalls nicht unbedingt flüssig sein. Z. B. bei der Untersuchung von Trinkwasser auf den Gehalt von freiem Chlor kann das Startreagenz auch in Tablettenform der Probe zugesetzt werden.

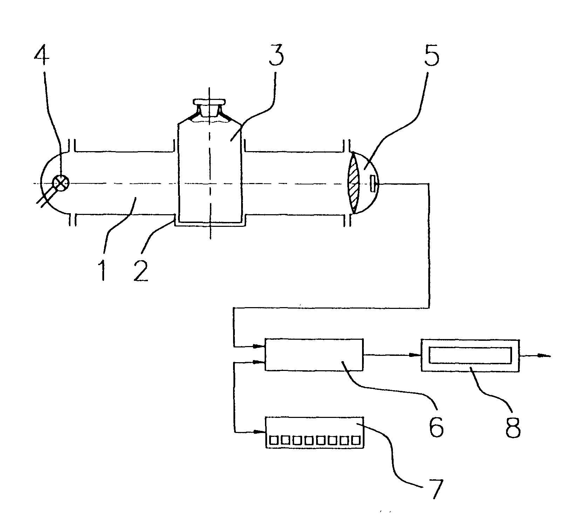

## Patentansprüche

1. Verfahren zur qualitativen und/oder quantitativen Bewertung einer Flüssigkeit als erstes Reagenz, wobei dieses erste Reagenz mit einer neutralen Flüssigkeit vermischt sein kann, bei dem das erste Reagenz beim Zusammentreffen mit einem zweiten Reagenz oder nach dem Zusetzen eines speziellen Startreagenz unter Änderung der Farbe chemisch und/oder biologisch reagiert und zu einer Änderung der Extinktion oder Transmission einer Lichtstrahlung beim Durchdringen der Reagenzien führt und die Änderung der Farbintensität und/oder die Geschwindigkeit der Farbänderung ermittelt und zur Auswertung mit Referenzwerten verglichen wird, wobei die Lichtstrahlung eine Bandbreite der Wellenlänge zwischen 50 und 150 nm innerhalb des Wellenbereiches der sich ausbildenden Färbung aufweist und die Extinktion vor oder kurz nach dem Zusammentreffen der beiden Reagenzien bzw. nach dem Zusetzen des speziellen Startreagenz und danach kontinuierlich oder diskontinuierlich bestimmt wird.

2. Verfahren nach Anspruch 1, **dadurch gekennzeichnet, daß** die Änderung der Extinktion bestimmt wird bis die Änderung mindestens nahezu den Wert Null angenommen hat.

3. Verfahren nach Anspruch 1, **dadurch gekennzeichnet, daß** als Lichtstrahlung eine gepulste Lichtstrahlung durch die Probe gesendet wird und der Extinktionswert bei jedem Lichtpuls oder einem Mehrfachen von Lichtpulsen erfaßt wird.

4. Verfahren nach einem der Ansprüche 1 bis 3, **dadurch gekennzeichnet, daß** die Extinktionswerte analog erfaßt werden und nach Umwandlung in eine digitale Form zur Auswertung genutzt werden.

5. Einrichtung zur qualitativen und/oder quantitativen Bewertung einer Flüssigkeit nach einem Verfahren entsprechend einem der Ansprüche 1 bis 4, **gekennzeichnet durch** einen Lichtschacht (1), der an einem Ende als Lichtquelle (4) eine Leuchtdiode aufweist, die ein Licht mit einer Bandbreite der Wellenlängen von maximal 150 nm aussendet, und einem Lichtsensor (5) am anderen Ende sowie einem Meßschacht (2) zwischen den beiden Elementen, einem Probenbehälter (3) für die Aufnahme der zu prüfenden Flüssigkeit, der paßgenau in den Meßschacht (2) eingebracht werden kann, einer elektronischen Meßwerterfassungseinheit, die dem Lichtsensor (5) nachgeschaltet ist und in der die analogen Meßwerte der Extinktion in digitale Werte gewandelt und bei Bedarf speichert werden, und einer Auswerteeinheit, in der die Referenzwerte für die spezifischen Wellenlängen der Lichtstrahlung gespeichert sind, mit denen die ermittelten Extinktionswerte der aktuellen Probe zur Auswertung verglichen werden können.

6. Einrichtung zur qualitativen und/oder quantitativen Bewertung einer Flüssigkeit nach einem Verfahren entsprechend einem der Ansprüche 1 bis 4, **gekennzeichnet durch** einen Lichtschacht (1), der an einem Ende eine breitbandige Lichtquelle (4) und am anderen Ende einen Lichtsensor (5) aufweist, der einen maximalen Meßbereich mit einer Bandbreite der erfaßbaren Wellenlängen von weniger als 150 nm aufweist, sowie einem Meßschacht (2) zwischen den beiden Elementen, einem Probenbehälter (3) für die Aufnahme der zu prüfenden Flüssigkeit, der paßgenau in den Meßschacht (2) eingebracht werden kann, einer elektronischen Meßwerterfassungseinheit, die dem Lichtsensor (5) nachgeschaltet ist und in der die analogen Meßwerte der Extinktion in digitale Werte gewandelt und bei Bedarf gespeichert werden, und einer Auswerteeinheit, in der die Referenzwerte für die spezifischen vom Lichtsensor erfaßten Wellenlängen gespeichert sind, mit denen die ermittelten Extinktionswerte der aktuellen Probe zur Auswertung verglichen werden können.

7. Einrichtung nach Anspruch 5 oder 6, **dadurch gekennzeichnet, daß** parallel neben der gekennzeichneten Einrichtung die jeweils andere Einrichtung nach Anspruch 5 bzw. 6 vorhanden ist und die Einrichtungen alternativ betrieben werden können.

8. Einrichtung nach einem der Ansprüche 5 bis 7, **dadurch gekennzeichnet, daß** das Ergebnis der Auswertung von der Auswerteeinheit auf einem Display und/oder an eine anderweitige Baueinheit, vorzugsweise einen elektronischen Rechner oder an einen Drucker, ausgegeben werden kann.

9. Einrichtung nach Anspruch 8, **dadurch gekennzeichnet, daß** in der Auswerteeinheit eine Vielzahl von Referenzwerten für unterschiedliche erste Reagenzien gespeichert sind, die wahlweise als Vergleichswerte aktiviert werden können.

## Claims

1. Method for the qualitative and/or quantitative assessment of a liquid as a first reagent, wherein this first reagent may be mixed with a neutral liquid, in which, when combined with a second reagent or after the addition of a special starting reagent, this first reagent reacts chemically and/or biologically with a colour change and leads to a change in the extinction or transmission of light radiation as it passes through the reagents, and the change in the colour intensity and/or the rate of the colour change is ascertained and compared with reference values for evaluation, wherein the light radiation has a wavelength bandwidth between 50 and 150 nm within the wave range of the coloration which is formed, and the extinction is determined before or shortly after the two reagents are combined, or after the special starting reagent is added, and continuously or discontinuously thereafter.

2. Method according to Claim 1, **characterized in that** the change in the extinction is determined until the change has at least approximately reached the value zero.

3. Method according to Claim 1, **characterized in that** pulsed light radiation is transmitted through the sample as the light radiation, and the extinction value is detected at each light pulse or a multiple of light pulses.

4. Method according to one of Claims 1 to 3, **characterized in that** the extinction values are detected in analogue mode, and are used for evaluation after conversion to a digital form.

5. Device for the qualitative and/or quantitative assessment of a liquid according to a method corresponding to one of Claims 1 to 4, **characterized by** a light shaft (1) which has a light-emitting diode at one end as a light source (4), which emits light with a wavelength bandwidth of at most 150 nm, and a light sensor (5) at the other end as well as a measurement shaft (2) between the two elements, a sample container (3) which is intended to hold the liquid to be tested and can be fitted accurately into the measurement shaft (2), an electronic measured-value detection unit, which is connected downstream of the light sensor (5) and in which the analogue measured values of the extinction are converted into digital values, and are stored if required, and an evaluation unit which stores the reference values for the specific wavelengths of the light radiation, with which the ascertained extinction values of the current sample can be compared for evaluation.

6. Device for the qualitative and/or quantitative assessment of a liquid according to a method corresponding to one of Claims 1 to 4, **characterized by** a light shaft (1) which has a broadband light source (4) at one end and, at the other end, a light sensor (5) which has a maximum measurement range with a detectable-wavelength bandwidth of less than 150 nm, as well as a measurement shaft (2) between the two elements, a sample container (3) which is intended to hold the liquid to be tested and can be fitted accurately into the measurement shaft (2), an electronic measured-value detection unit, which is connected downstream of the light sensor (5) and in which the analogue measured values of the extinction are converted into digital values, and are stored if required, and an evaluation unit which stores the reference values for the specific wavelengths detected by the light sensor, with which the ascertained extinction values of the current sample can be compared for evaluation.

7. Device according to Claim 5 or 6, **characterized in that** the other device according to Claim 5 or 6, respectively, is in each case present in parallel next to the characterized device, and the devices can be operated alternately.

8. Device according to one of Claims 5 to 7, **characterized in that** the result of the evaluation by the evaluation unit can be output on a display and/or to another kind of component, preferably an electronic computer or to a printer.

9. Device according to Claim 8, **characterized in that** the evaluation unit stores a plurality of reference values for various first reagents, which can be selectively activated as comparison values.

## Revendications

1. Procédé pour l'évaluation qualitative et/ou quantitative d'un liquide en tant que premier réactif, ce premier réactif pouvant être mélangé avec un liquide neutre, avec lequel le premier réactif lors de la rencontre d'un deuxième réactif ou après l'ajout d'un réagent inducteur spécial réagit de manière chimique et/ou biologique en modifiant la couleur et provoque une modification de l'extinction ou de la transmission d'un rayon lumineux lors du passage à travers des réactifs et la modification de l'intensité de la coloration et/ou la vitesse de variation de la couleur est déterminée et comparée avec des valeurs de référence en vue de son évaluation, le rayon lumineux présentant une bande passante de longueur d'onde entre 50 et 150 nm à l'intérieur de la plage d'ondes de la coloration qui se forme et l'extinction étant déterminée ensuite continuellement ou de manière discontinue avant ou immédiatement après la rencontre des deux réactifs ou après l'ajout du réagent inducteur spécial.

2. Procédé selon la revendication 1, **caractérisé en ce que** la modification de l'extinction est déterminée jusqu'à ce que la modification atteigne une valeur pratiquement égale à zéro.

3. Procédé selon la revendication 1, **caractérisé en ce que** le rayon lumineux émis à travers l'échantillon est un rayon lumineux pulsé et la valeur d'extinction est détectée à chaque impulsion lumineuse ou un multiple d'impulsions lumineuses.

4. Procédé selon l'une des revendications 1 à 3, **caractérisé en ce que** les valeurs d'extinction sont détectées de manière analogique et qu'elles sont utilisées pour évaluation après conversion sous forme numérique.

5. Dispositif pour l'évaluation qualitative et/ou quantitative d'un liquide selon l'un des procédés conformes à l'une des revendications 1 à 4, **caractérisé par** un puits de lumière (1) qui présente à une extrémité une diode électroluminescente faisant office de source de lumière (4), laquelle émet une lumière ayant une largeur de bande de longueur d'onde de 150 nm au maximum, et un capteur de lumière (5) à l'autre extrémité ainsi qu'un puits de mesure (2) entre les deux éléments, un récipient à échantillon (3) destiné à recevoir le liquide à contrôler, lequel vient parfaitement se loger dans le puits de mesure (2), un module électronique d'acquisition de valeur mesurée, qui est branché en amont du capteur de lumière (5) et dans lequel les valeurs de mesure analogiques de l'extinction sont converties en valeurs numériques et éventuellement mémorisées, et un module d'évaluation dans lequel sont mémorisées les valeurs de référence pour les longueurs d'onde spécifiques du rayon lumineux avec lesquelles peuvent être comparées les valeurs d'extinction déterminées de l'échantillon courant en vue de leur évaluation.

6. Dispositif pour l'évaluation qualitative et/ou quantitative d'un liquide selon l'un des procédés conformes à l'une des revendications 1 à 4, **caractérisé par** un puits de lumière (1) qui présente à une extrémité une source de lumière à large bande (4) et à l'autre extrémité un capteur de lumière (5), lequel présente une plage de mesure maximale ayant une bande passante de la longueur d'inde détectable de moins de 150 nm, ainsi qu'un puits de mesure (2) entre les deux éléments, un récipient à échantillon (3) destiné à recevoir le liquide à controler, lequel vient parfaitement se loger dans le puits de mesure (2), un module électronique d'acquisition de valeur mesurée, qui est branché en amont du capteur de lumière (5) et dans lequel les valeurs de mesure analogiques de l'extinction sont converties en valeurs numériques et éventuellement mémorisées, et un module d'évaluation dans lequel sont mémorisées les valeurs de référence pour les longueurs d'onde spécifiques captées par le capteur de lumière, avec lesquelles peuvent être comparées les valeurs d'extinction déterminées de l'échantillon courant en vue de leur évaluation.

7. Dispositif selon la revendication 5 ou 6, **caractérisé en ce que** l'autre dispositif selon la revendication 5 ou 6 est présent en parallèle en plus du dispositif caractérisé et les dispositifs peuvent être utilisés en alternance.

8. Dispositif selon l'une des revendications 5 à 7, **caractérisé en ce que** le résultat de l'évaluation du module d'évaluation peut être sorti sur un afficheur et/ou sur un autre type d'élément, de préférence un calculateur électronique ou sur une imprimante.

9. Dispositif selon la revendication 8, **caractérisé en ce qu'**une pluralité de valeurs de référence pour différents premiers réactifs sont mémorisées dans le module d'évaluation, lesquelles peuvent être activées au choix comme valeurs de comparaison.
